# EUROPEAN PATENT APPLICATION

(11) **EP 4 640 173 A2**
(43) Date of publication of application: **29.10.2025**
(21) Application number: 25199709.4
(22) Date of filing: 19.10.2020
(51) Int. Cl.: A61B 18/00

(54) **CARDIAC MAPPING CATHETER WITH SQUARE-SPACED ELECTRODES**

(30) Priority: 23.10.2019 US 201962924832 P; 11.09.2020 US 202017018111
(62) Divisional of application: 20807113.4
(71) Applicant: Biosense Webster (Israel) Ltd., 2066717 Yokneam (IL)
(72) Inventor: BASU, Shubhayu, Irwindale, 91706 (US); TOBEY, Dustin R., Irwindale, 91706 (US); FOLEY, John J., Irwindale, 91706 (US); FUENTES-ORTEGA, Cesar, Irwindale, 91706 (US)
(74) Representative: Carpmaels & Ransford LLP

(57) **Abstract**

An apparatus includes a catheter shaft assembly and an end effector. The end effector includes a plurality of strips and a plurality of electrodes. The strips are configured to fit within an outer sheath of the catheter shaft assembly in a first configuration. The strips are configured to expand outwardly away from a longitudinal axis defined by the catheter shaft assembly in a second configuration when exposed distally relative to the distal end of the outer sheath. The electrodes are positioned on at least some of the strips. The electrodes are positioned relative to each other such that groups of four of the electrodes define a substantially square configuration.

## Description

### PRIORITY

This application claims priority to U.S. Provisional Pat. App. No. 62/924,832, entitled "Cardiac Mapping Catheter with Square-Spaced Electrodes," filed October 23, 2019, the disclosure of which is incorporated by reference herein in its entirety.

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., radiofrequency (RF) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more RF electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The electrodes may be placed in contact with cardiac tissue or other vascular tissue and then activated with RF energy to thereby ablate the contacted tissue. In some cases, the electrodes may be bipolar. In some other cases, a monopolar electrode may be used in conjunction with a ground pad or other reference electrode that is in contact with the patient.

Examples of ablation catheters are described in U.S. Pub. No. 2013/0030426, entitled "Integrated Ablation System using Catheter with Multiple Irrigation Lumens," published January 31, 2013, the disclosure of which is incorporated by reference herein, in its entirety; U.S. Pub. No. 2017/0312022, entitled "Irrigated Balloon Catheter with Flexible Circuit Electrode Assembly," published November 2, 2017, the disclosure of which is incorporated by reference herein, in its entirety; U.S. Pub. No. 2018/0071017, entitled "Ablation Catheter with a Flexible Printed Circuit Board," published March 15, 2018, the disclosure of which is incorporated by reference herein, in its entirety; U.S. Pub. No. 2018/0056038, entitled "Catheter with Bipole Electrode Spacer and Related Methods," published March 1, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; U.S. Pat. No. 8,956,353, entitled "Electrode Irrigation Using Micro-Jets," issued February 17, 2015, the disclosure of which is incorporated by reference herein, in its entirety; and U.S. Pat. No. 9,801,585, entitled "Electrocardiogram Noise Reduction," issued October 31, 2017, the disclosure of which is incorporated by reference herein, in its entirety.

Some catheter ablation procedures may be performed after using electrophysiology (EP) mapping to identify tissue regions that should be targeted for ablation. Such EP mapping may include the use of sensing electrodes on a catheter (e.g., the same catheter that is used to perform the ablation or a dedicated mapping catheter). Such sensing electrodes may monitor electrical signals emanating from conductive endocardial tissues to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998, the disclosure of which is incorporated by reference herein, in its entirety. Examples of EP mapping catheters are described in U.S. Pat. No. 9,907,480, entitled "Catheter Spine Assembly with Closely-Spaced Bipole Microelectrodes," issued March 6, 2018, the disclosure of which is incorporated by reference herein, in its entirety; U.S. Pat. No. 10,130,422, entitled "Catheter with Soft Distal Tip for Mapping and Ablating Tubular Region," issued November 20, 2018, the disclosure of which is incorporated by reference herein, in its entirety; and U.S. Pub. No. 2018/0056038, entitled "Catheter with Bipole Electrode Spacer and Related Methods," published March 1, 2018, the disclosure of which is incorporated by reference herein, in its entirety.

In addition to using EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 9,480,416, entitled "Signal Transmission Using Catheter Braid Wires," issued November 1, 2016, the disclosure of which is incorporated by reference herein, in its entirety; and various other references that are cited herein.

While several catheter systems and methods have been made and used, it is believed that no one prior to the inventors has made or used the invention described in the appended claims.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2A depicts a perspective view of a distal portion of the catheter of FIG. 1, with an end effector of the catheter in a proximal position relative to an outer sheath of the catheter;
FIG. 2B depicts a perspective view of the distal portion of FIG. 2A, with the end effector in a distal position relative to the outer sheath, and with the end effector in an outwardly expanded state;
FIG. 3 depicts a side elevation view of the end effector of FIG. 2A in the outwardly expanded state;
FIG. 4 depicts a distal end view of the end effector of FIG. 2A in the outwardly expanded state;
FIG. 5 depicts a perspective view of an electrode assembly of the end effector of FIG. 2A;
FIG. 6 depicts a perspective view of a distal portion of the electrode assembly of FIG. 5;
FIG. 7 depicts a perspective view of a proximal portion of the electrode assembly of FIG. 5;
FIG. 8 depicts an enlarged plan view of a portion of the electrode assembly of FIG. 5;
FIG. 9 depicts a perspective view of an example of an alternative end effector that may be incorporated into the catheter of FIG. 1, with the alternative end effector in an expanded state;
FIG. 10 depicts a perspective view of an arrangement of alternative electrode assemblies that may be incorporated into an alternative end effector of the catheter of FIG. 1;
FIG. 11 depicts a distal end view of the arrangement of electrode assemblies of FIG. 10;
FIG. 12 depicts an enlarged plan view of a portion of one of the electrode assemblies of the arrangement of electrode assemblies of FIG. 10;
FIG. 13 depicts a perspective view of an example of another alternative electrode assembly that may be incorporated into an alternative end effector of the catheter of FIG. 1;
FIG. 14 depicts a top plan view of the electrode assembly of FIG. 13;
FIG. 15 depicts a perspective view of a distal portion of the electrode assembly of FIG. 13; and
FIG. 16 depicts a perspective view of a proximal portion of the electrode assembly of FIG. 13.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "generally," "substantially," "about," or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

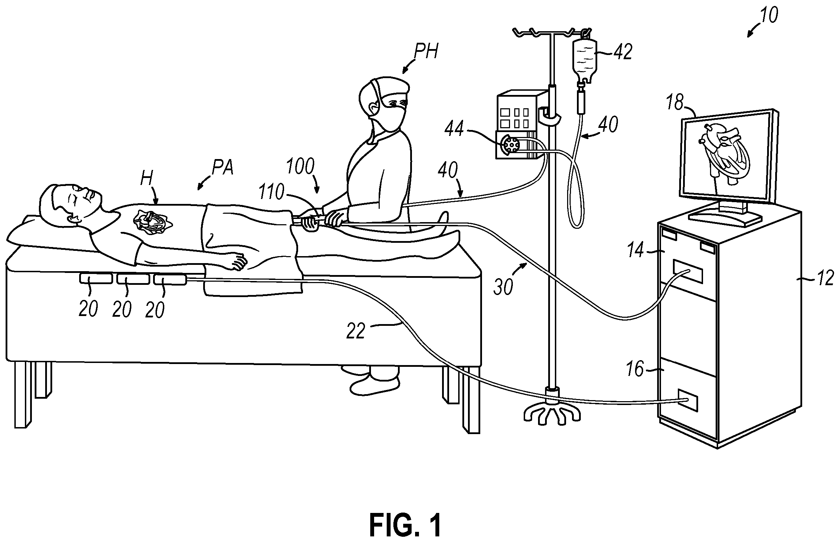

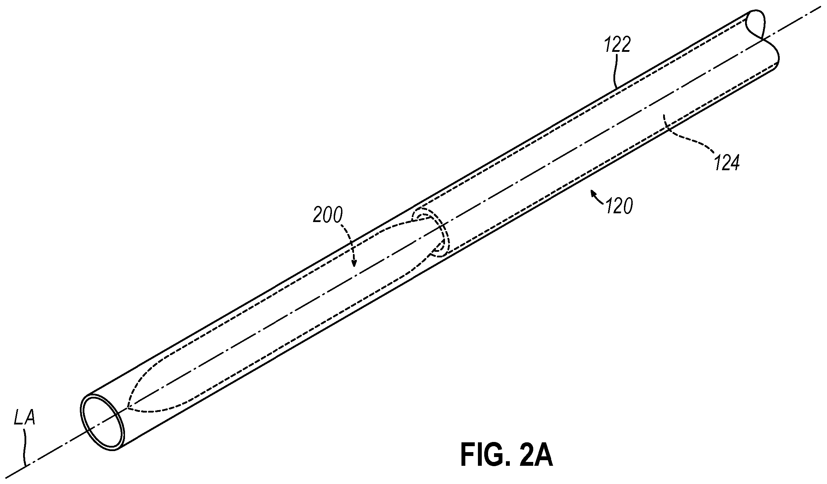

FIG. 1 shows an example of a medical procedure and associated components of a cardiac mapping or ablation system. In particular, FIG. 1 shows a physician (PH) grasping a handle (110) of a catheter assembly (100), with an end effector (200) of a flexible catheter (120) (shown in FIGS. 2A-4 but not shown in FIG. 1) of catheter assembly (100) disposed in a patient (PA) to map or ablate tissue in or near the heart (H) of the patient (PA). As shown in FIGS. 2A-2B, catheter (120) includes an outer sheath (122), a first inner shaft (124), and a second inner shaft (126). First inner shaft (124) is coaxially and slidably disposed within outer sheath (122). Second inner shaft (126) is coaxially disposed within first inner shaft (124). In some versions, second inner shaft (126) is operable to slide in relation to first inner shaft (124), though this is optional. End effector (200) is positioned at the distal end of first inner shaft (124) and encompasses the distal portion of second inner shaft (126). Catheter assembly (100) is coupled with a guidance and drive system (10) via a cable (30). Catheter assembly (100) is also coupled with a fluid source (42) via a fluid conduit (40), though this is optional. A set of field generators (20) are positioned underneath the patient (PA) and are also coupled with guidance and drive system (10) via a cable (22).

Guidance and drive system (10) of the present example includes a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via electrode pairs (230) of end effector (200) as described in greater detail below. Console (12) includes a processor (not shown) that processes such EP mapping signals and thereby provides EP mapping as is known in the art. In addition, or in the alternative, first driver module (14) may be operable to provide RF power to ablation electrodes (not shown) of end effector (200) to thereby ablate tissue. In some versions, first driver module (14) is also operable to receive position indicative signals from one or more position sensors (127) in end effector (200), as will be described in greater detail below. In such versions, the processor of console (12) is also operable to process the position indicative signals from the position sensor (127) to thereby determine the position of the end effector (200) of catheter (120) within the patient (PA).

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from the position sensor of end effector (200). For instance, as end effector (200) of catheter (120) moves within the patient (PA), the corresponding position data from position sensor (127) may cause the processor of console (12) to update the patient anatomy views in display (18) in real time to depict the regions of patient anatomy around end effector (200) as end effector (200) moves within the patient (PA). Moreover, the processor of console (12) may drive display (18) to show locations of aberrant conductive tissue sites, as detected via EP mapping with end effector (200). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (200) on the images of the patient's anatomy, such as by superimposing an illuminated dot, a crosshair, a graphical representation of end effector (200), or some other form of visual indication. Such a superimposed visual indication may also move within the images of the patient anatomy on display (18) in real time as the physician moves end effector (200) within the patient (PA), thereby providing real-time visual feedback to the operator about the position of end effector (200) within the patient (PA) as end effector (200) moves within the patient (PA). The images provided through display (18) may thus effectively provide a video tracking the position of end effector (200) within a patient (PA), without necessarily having any optical instrumentation (i.e., cameras) viewing end effector (200). In the same view, display (18) may simultaneously visually indicate the locations of aberrant conductive tissue sites detected through the EP mapping as described herein. The physician (PH) may thus view display (18) to observe the real time positioning of end effector (200) in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). In some variations, conduit (40), fluid source (42), and pump (44) are omitted entirely. In versions where these components are included, end effector (200) may be configured to communicate irrigation fluid from fluid source (42) to the target site in the patient. Such irrigation may be provided in accordance with the teachings of any of the various patent references cited herein; or in any other suitable fashion as will be apparent to those skilled in the art in view of the teachings herein.

### II. Example of an End Effector

To the extent that cardiac EP mapping end effectors are already known in the art, such EP mapping end effectors may generally fit in only one of two different categories. One category is for EP mapping end effectors that are particularly configured for use in the chambers of a heart (H), where the end effector will be pressed against the walls defining the chambers of the heart (H) in order to pick up electrocardiogram signals from the tissue. Another category is for EP mapping end effectors that are particularly configured for use in the pulmonary vein, where the end effector will be pressed against the wall of the pulmonary vein in order to pick up electrocardiogram signals from the tissue. Those skilled in the art will recognize that the geometry of the wall of the pulmonary vein is substantially different from the walls defining the chambers of the heart (H). For instance, the wall of the pulmonary vein has a generally circular cross-section, with a relatively small radius of curvature. By contrast, the walls defining the chambers of the heart (H) may have generally flat regions and curved regions with a radius of curvature that is larger than the radius of curvature associated with the pulmonary vein.

Thus, due to the differences in geometries of the pulmonary vein and the walls defining the chambers of the heart (H), an EP mapping end effector that is specifically configured for use in a pulmonary vein may not be suitable for use in the chambers of the heart (H). Similarly, an EP mapping end effector that is specifically configured for use in the chambers of the heart (H) may not be suitable for use in the pulmonary vein. It may therefore be desirable to provide an EP mapping end effector that is suitable for use in both the pulmonary vein and in the chambers of the heart (H). An example of such an end effector (200) is described in greater detail below.

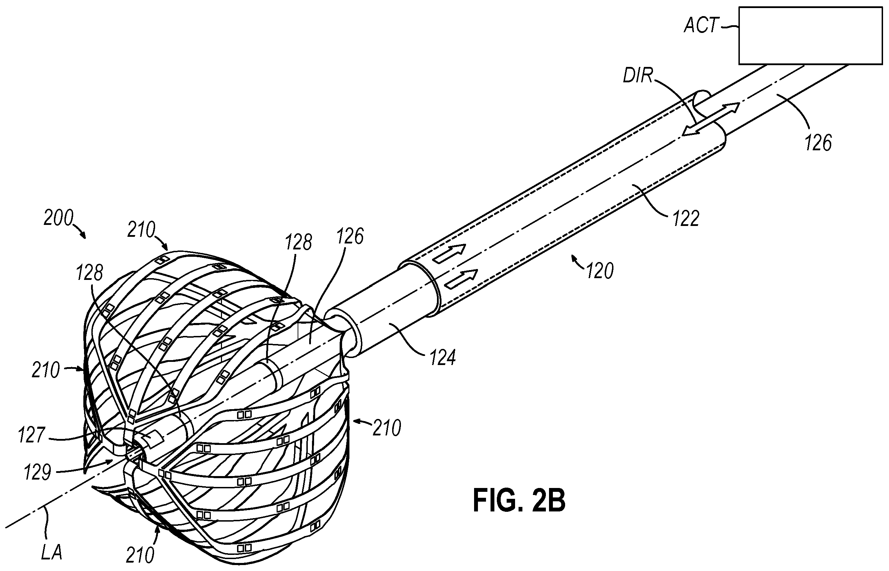

FIGS. 2B-4 show end effector (200) in greater detail. As shown, end effector (200) includes a set of electrode assemblies (210) that are compressible to fit within an outer sheath (122). As noted above, end effector (200) is mounted to first inner shaft (124), which is internal to outer sheath (122) and is slidably disposed relative to outer sheath (122). FIG. 2A shows a state in which end effector (200) is retracted proximally relative to outer sheath (122), such that end effector (200) is proximal to the distal end of outer sheath (122). In this state, end effector (200) deformably conforms to the cylindraceous interior of outer sheath (122). Catheter (120) and end effector (200) may be in the state shown in FIG. 2A when catheter (120) is introduced into the body of the patient (PA); and during transit from the insertion site to the targeted cardiovascular region within the patient (PA).

FIG. 2B shows a state in which end effector (200) is advanced distally relative to outer sheath (122), such that end effector (200) is distal to the distal end of outer sheath (122). As shown in FIG. 2B, electrode assemblies (210) are resiliently biased toward an outwardly expanded configuration, such that electrode assemblies (210) bow outwardly away from the longitudinal axis (LA) of catheter (120). End effector (200) thus presents a generally spherical configuration in this state.

In some versions, in order to transition between the state shown in FIG. 2A and the state shown in FIG. 2B, first inner shaft (124) remains longitudinally stationary relative to handle (110) while outer sheath (122) translates longitudinally relative to handle (110) and relative to first inner shaft (124). In such versions, handle (110) or the proximal end of outer sheath (122) may include an actuator (ACT) that may be manipulated by the physician (PH) to drive outer (122) sheath longitudinally relative to handle (110) and relative to inner shaft (124). As another merely illustrative variation, in order to transition between the state shown in FIG. 2A and the state shown in FIG. 2B, outer sheath (122) remains longitudinally stationary relative to handle (110) while first inner shaft (124) translates longitudinally relative to handle (110) and relative to outer sheath (122). In such versions, handle (110) may include an actuator that may be manipulated by the physician (PH) to drive first inner shaft (124) longitudinally relative to handle (110) and relative to outer sheath (122).

In addition to (or instead of) electrode assemblies (210) being resiliently biased to assume the outwardly bowed configuration shown in FIGS. 2B-4, one inner shaft (124, 126) may translate relative to the other inner shaft (124, 126) in order to transition end effector (200) between a non-expanded state and an expanded state. For instance, to provide end effector (200) in a non-expanded state, second inner shaft (126) may be positioned further distally (relative to first inner shaft (124)) than the position shown in FIGS. 2B-4. In some such versions, first inner shaft (124) would then be advanced distally relative to second inner shaft (126) to translate the proximal portions (240) of electrode assemblies (210) distally relative to the distal portions (250) of electrode assemblies (210), while second inner shaft (126) remains stationary. This distal translation of proximal portions (240) relative to distal portions (250) would cause electrode assemblies (210) to buckle outwardly, thereby transitioning end effector (200) from the non-expanded state to the expanded state. As another merely illustrative variation, first inner shaft (124) may remain stationary as second inner shaft (126) is retracted proximally, thereby driving distal portions (250) proximally relative to proximal portions (240). This proximal translation of distal portions (250) relative to proximal portions (240) would cause electrode assemblies (210) to buckle outwardly, thereby transitioning end effector (200) from the non-expanded state to the expanded state. In either case, the foregoing movements may be reversed to return end effector (200) to the non-expanded state; and outer sheath (122) may be advanced over the non-expanded end effector (200) for withdrawal from the patient (PA) at the end of the procedure.

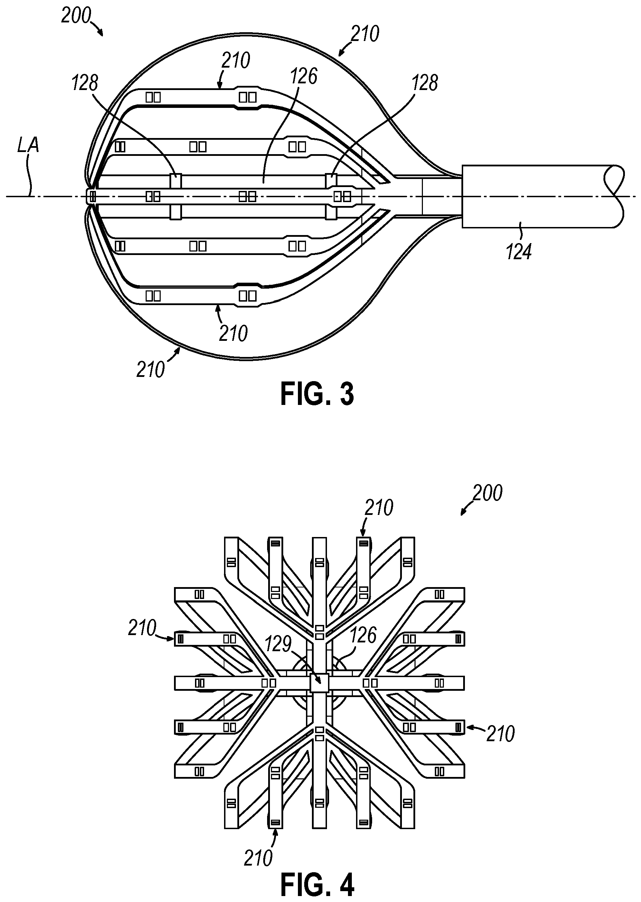

As best seen in FIG. 4, end effector (200) of the present example includes four electrode assemblies (210), which are angularly spaced apart from each other equidistantly about the longitudinal axis (LA) of catheter (120). Alternatively, any other suitable number of electrode assemblies (210) may be used, including but not limited to two electrode assemblies (210), three electrode assemblies (210), or more than four electrode assemblies (210).

End effector (200) of the present example further includes a position sensor (127) located near distal end (129) of second inner shaft (126). Position sensor (127) is operable to generate signals that are indicative of the position and orientation of end effector (200) within the patient (PA). By way of example only, position sensor (127) may be in the form of a wire coil or a plurality of wire coils (e.g., three orthogonal coils) that are configured to generate electrical signals in response to the presence of an alternating electromagnetic field generated by field generators (20). Position sensor (127) may be coupled with wire, a trace, or any other suitable electrical conduit along or otherwise through catheter (120), thereby enabling signals generated by position sensor (127) to be communicated back through electrical conduits (not shown) in catheter (120) to console (12). Console (12) may process the signals from position sensor (127) to identify the position of end effector (200) within the patient (PA). Other components and techniques that may be used to generate real-time position data associated with end effector (200) may include wireless triangulation, acoustic tracking, optical tracking, inertial tracking, and the like. In some versions, position sensor (127) may be omitted.

As described in greater detail below, electrode pairs (230) of each electrode assembly (210) are operable to provide EP mapping within the cardiac system of the patient (PA). As shown in FIGS. 2B-3, second inner shaft (126) includes a pair of reference electrodes (128) that are coaxially positioned about second inner shaft (126). Such reference electrodes (128) may be utilized in conjunction with electrode pairs (230) during an EP mapping procedure. For instance, reference electrodes (128) may be utilized to pick up reference potentials from blood or saline that passes through the interior of end effector (200) during an EP mapping procedure. Such reference potentials may be used to reduce noise or far field signals, as is known in the art. In the present example, since reference electrodes (128) are effectively contained within an interior defined by electrode assemblies (210), electrode assemblies (210) will prevent tissue from contacting reference electrodes (128) during use of end effector (200) in an EP mapping procedure; while still allowing blood and saline to flow freely through end effector (200) to reach reference electrodes (128).

As noted above, catheter assembly (100) of the present example is coupled with a fluid source (42) via a fluid conduit (40). A fluid conduit (not shown) extends along the length of catheter (120) and is operable to deliver irrigation fluid (e.g., saline) out through the open distal end (129) of second inner shaft (126). For instance, the fluid conduit may distally terminate at distal end (129). In addition, or in the alternative, second inner shaft (126) may incorporate one or more laterally oriented irrigation ports that are in communication with the fluid conduit. Such irrigation ports may be spaced apart along the region of length corresponding to the longitudinal position of end effector (200). In either case, the irrigation fluid may provide cooling, flushing, or other effects at end effector (200) during operation of end effector (200) within the patient (PH). Various suitable ways in which catheter assembly (100) may provide irrigation will be apparent to those skilled in the art. Alternatively, some variations of catheter assembly (100) may lack irrigation capabilities, such that conduit (40), fluid source (42), and pump (44) may be omitted.

In addition to the foregoing, end effector (200) and other aspects of catheter assembly (100) may be configured and operable in accordance with at least some of the teachings of U.S. Pub. No. 2018/0056038, the disclosure of which is incorporated by reference herein, in its entirety.

### III. Example of an Electrode Assembly

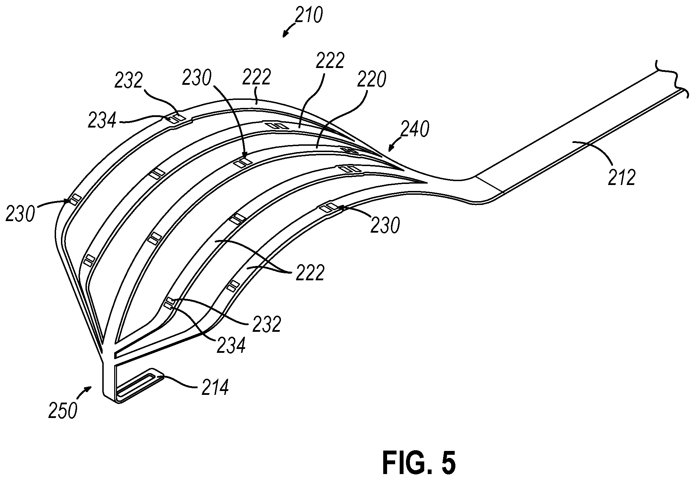

FIGS. 5-7 depicts a single electrode assembly (210) of end effector (200) in greater detail. Electrode or grid assembly (210) of this example includes a proximal end strip (212), a distal end strip (214), and a plurality of strips (220, 222) extending between strips (212, 214). Strips (220, 222) include a central strip (220) and a plurality of laterally positioned strips (222) positioned on each side of central strip (220). Proximal end strip (212) extends proximally into the interior of first inner shaft (124), thereby proximally anchoring each electrode assembly (210) in first inner shaft (124). Distal end strip (214) extends proximally into the interior of second inner shaft (126) and can be secured to a movable actuator (ACT) via second inner shaft (126), thereby securing each electrode assembly (210). As shown schematically in FIG. 2B, a movable actuator (ACT) may be secured to second inner shaft (126), such that actuator (ACT) may drive distal end strips (214) longitudinally. In other words, the actuator (ACT) can be moved along the longitudinal axis (LA) to allow the operator to change configurations of electrode assemblies (210), thereby changing the grid configurations of the electrodes (232, 234) on electrode assemblies (210). Thus, movement along the longitudinal axis (LA) in the directions shown by arrow (DIR) in FIG. 2B will cause each electrode assembly (210) to expand or contract continuously to define various radius of curvature (R) with respect to longitudinal axis (LA) for each electrode assembly (210). Each electrode assembly (210) therefore takes on a portion of a spheroidal shape depending on the movement of the actuator (ACT). Full extension of the actuator (ACT) will cause each electrode assembly (210) to flatten along the longitudinal axis (LA) for retrieval into the outer tubular member (122). In some versions, each electrode assembly (210) can be connected to independent actuators (ACT) so that only one of the actuators (ACT) may be needed to actuate a desired electrode assembly (210) while the other electrode assemblies (210) remain flat or in a different configuration depending on the location of the respective actuators (ACT) of the electrode assemblies (210). Alternatively, all electrode assemblies (210) may be coupled with just one actuator (ACT). For clarity, additional actuators (ACT) are not shown in the remainder of the drawings to provide clarity on the spatial arrangement of the electrode grids (210). Various other suitable ways in which end strips (212, 214) may be secured relative to the respective shafts (124, 126), and driven, will be apparent to those skilled in the art in view of the teachings herein.

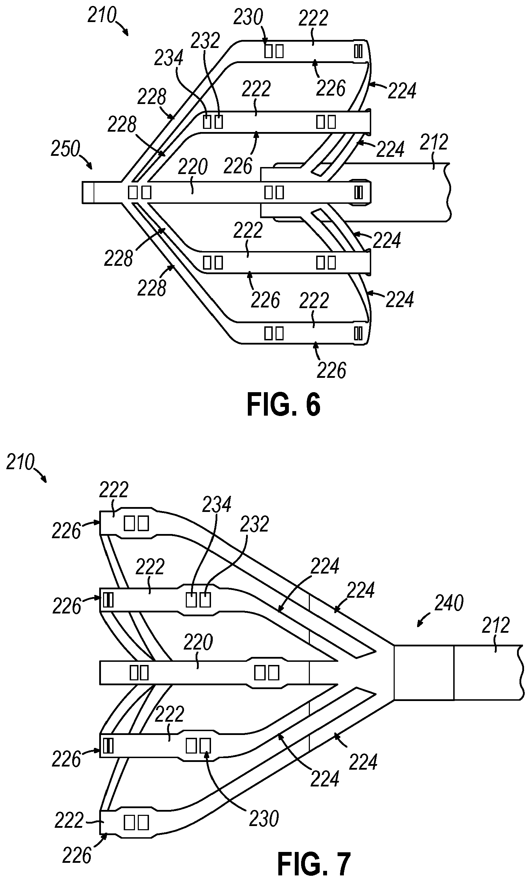

In the present example, there are two strips (222) on each side of central strip (220), though any other suitable number of laterally positioned strips (222) may be provided. As best seen in FIGS. 6-7, each strip (222) includes a proximal segment (224), an intermediate segment (226), and a distal segment (228). Proximal segments (224) diverge outwardly, away from central strip (220). Intermediate segments (226) are parallel with central strip (220). Distal segments (228) converge inwardly, toward central strip (220). In the present example, distal segments (228) are positioned closer to each other than proximal segments (224) are positioned relative to each other. Alternatively, any other suitable spacing may be utilized. For example, the spacing of the mapping electrodes can be arranged to conform to the arrangements shown and described in U.S. Pat. App. No. 16/820,848, entitled "Electrode Configurations for Diagnosis of Arrhythmias," filed March 18, 2019, the disclosure of which is incorporated by reference herein, in its entirety. With strips (220, 222) being spaced apart from each other, and with electrode assemblies (210) being spaced apart from each other, end effector (200) is configured to allow blood to flow through end effector (200) during use of end effector (200) (e.g., when end effector (200) is in an expanded state within a chamber of the heart (H), the pulmonary vein, etc.). As noted above, such flowing blood may also reach reference electrodes (128) on second inner shaft (126) during use of end effector (200).

Each strip (220, 222) has several electrode pairs (230) positioned along the length of strip (220, 222). Each electrode pair (230) includes a proximal electrode (232) and a distal electrode (234). Electrodes (232, 234) of each pair (230) are operable to provide bipolar EP mapping by picking up electrocardiogram signals from tissue as is known in the art. In some other variations (e.g., as described in the context of further examples below), a single electrode is positioned where each pair (230) is positioned on electrode assembly (210). In either case, signals picked up by electrodes (232, 234) may be communicated back through electrical conduits (not shown) in catheter (120) to console (12), which may process the signals to provide EP mapping to thereby identify locations of aberrant electrical activity within the cardiac anatomy. This may in turn allow the physician (PH) to identify the most appropriate regions of cardiac tissue to ablate (e.g., with RF energy, cryoablation, etc.), to thereby prevent or at least reduce the communication of aberrant electrical activity across the cardiac tissue.

Each electrode assembly (210) of the present example is in the form of a flex circuit. For instance, each electrode assembly (210) may include a substrate formed of polyimide, polyether ether ketone, or any other suitable flex circuit substrate; with electrodes (232, 234) and associated conductive traces printed on the substrate. In versions where each electrode assembly (210) is resiliently biased to bow outwardly to provide end effector (200) in an expanded configuration, one or more resilient elements may be integrated into teach electrode assembly (210). By way of example only, one or more strips of resilient material (e.g., nitinol, etc.) may be integrated into teach electrode assembly (210).

By way of example only, electrodes (128, 232, 234) may be formed of platinum, gold, or any other suitable material. Electrodes (128, 232, 234) may include various coatings, if desired. For instance, electrode pairs (230) may include a coating that is selected to improve the signal-to-noise ratio of signals from electrode pairs (230). Such coatings may include, but need not be limited to, iridium oxide (IrOx) coating, poly(3,4-ethylenedioxythiophene) (PEDOT) coating, Electrodeposited Iridium Oxide (EIROF) coating, Platinum Iridium (PtIr) coating, or any other suitable coating. Various suitable kinds of coatings that may be used for electrodes (128, 232, 234) will be apparent to those skilled in the art in view of the teachings herein.

In the present example electrodes (232, 234) may be provided on the substrate of electrode assembly (210) as a thin film through a physical vapor deposition (PVD) process. way of example only, such a PVD process may be carried out in accordance with at least some of the teachings of International Patent Pub. No. WO 2015/117908, entitled "Medical Device for Ablating Tissue Cells and System Comprising a Device of This Type," published August 13, 2015, the disclosure of which is incorporated by reference herein, in its entirety; at least some of the teachings of German Patent Pub. No. 102017130152, entitled "Method for Operating a Multi-Layer Structure," published January 3, 2019, the disclosure of which is incorporated by reference herein, in its entirety; or at least some of the teachings of U.S. Pat. No. 10,061,198, entitled "Method for Producing a Medical Device or a Device with Structure Elements, Method for Modifying the Surface of a Medical Device or of a Device with Structure Elements, Medical Device and Laminated Composite with a Substrate," published August 28, 2018, the disclosure of which is incorporated by reference herein, in its entirety. Other methods may also be employed to provide electrodes (232, 234), conductive traces, or other circuit components on electrode assemblies (210), including but not limited to sputter deposition, chemical vapor deposition (CVD), thermal deposition, etc. Regardless of the methods used, each electrode assembly (210) may ultimately constitute a flex circuit.

As noted above, each electrode assembly (210) of the present example is flexible enough to compress within outer sheath (122) as shown in FIG. 2A; yet is resiliently biased toward the outwardly bowed configuration shown in FIG. 2B. In some variations, this resilience is provided by the material forming the substrate of each electrode assembly (210). In some other variations, one or more resilient features (not shown) are added to the substrate of each electrode assembly (210) to impart the resilient bias. By way of example only, one or more nitinol strips or other nitinol structures may be applied to the substrate of each electrode assembly (210), such as along any one or more of strips (220, 222). Such nitinol strips may be applied using the vapor deposition process or other manufacturing techniques noted above.

As noted above, each electrode assembly (210) is flexible, such that end effector (200) may conform to the contours and other surface geometry of cardiac tissue when end effector (200) is pressed against cardiac tissue (e.g., within a chamber of the heart (H), within the pulmonary vein, etc.). The deformation of end effector (200) may promote full contact between electrode pairs (230) and cardiac tissue. Such contact may be further promoted by providing a substantial number of electrode pairs (230) on each electrode assembly (210). Having a substantial number of electrode pairs (230) may enable end effector (200) to provide high density EP mapping through all four chambers of the heart (H), within the pulmonary vein, etc., as several pairs of electrode pairs (230) can provide electrocardiogram signal sensing at multiple regions of cardiac tissue simultaneously.

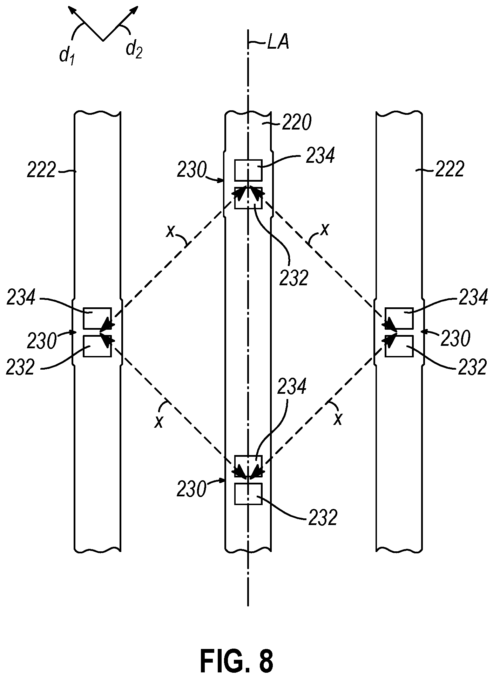

When end effector (200) is pressed against cardiac tissue to pick up electrocardiogram signals, electrode pairs (230) are positioned to contact the tissue in a substantially square configuration, with substantially square spacing. In this context, the terms "substantially square configuration" and "substantially square spacing" should be read to include the electrode pairs (230) being spaced apart from each other by the same distance in two perpendicular directions. An example of this substantially square spacing is shown in FIG. 8, where the two perpendicular direction are shown as being along a first dimension (d₁) and a second dimension (d₂). As shown, an electrode pair (230) on one strip (222) is spaced apart along the first dimension (d₁) from a first electrode pair (230) on strip (220) by a distance (x); and along the second dimension (d₂) from a second electrode pair (230) on strip (220) by the same distance (x). Similarly, an electrode pair (230) on another strip (222) is spaced apart along the first dimension (d₁) from the second electrode pair (230) on strip (220) by that same distance (x); and along the second dimension (d₂) from the first electrode pair (230) on strip (220) by the same distance (x). These four electrode pairs (230) are thus arranged in a substantially square-shaped arrangement, with substantially square spacing, spaced equidistantly by the distance (x). This same substantially square spacing may apply to all electrode pairs (230) on each electrode assembly (210). It is noted that the electrode arrangement is not limited to the substantially square-spacing arrangement and the electrodes can be arranged to conform to the arrangements shown and described in U.S. Pat. App. No. 16/820,848, entitled "Electrode Configurations for Diagnosis of Arrhythmias," filed March 18, 2019, the disclosure of which is incorporated by reference herein, in its entirety.

In the present example, the dimensions (d₁, d₂) of spacing between electrode pairs (230) are obliquely oriented relative to the longitudinal axis (LA) of end effector (200). For instance, the dimensions (d₁, d₂) of spacing between electrode pairs (230) may be oriented relative to the longitudinal axis (LA) of end effector (200) at an angle of approximately 45°. In some other versions, the first dimension (d₁) of spacing between electrode pairs (230) is parallel to the longitudinal axis (LA) of end effector (200); while the second dimension (d₂) of spacing between electrode pairs (230) is perpendicular to the longitudinal axis (LA) of end effector (200).

As noted above and as shown in FIGS. 2B-7, strips (220, 222) will have a generally curved configuration until something urges them to deform. For instance, as shown in FIG. 2A, outer sheath (122) may deform strips (220, 222) in order for strips (220, 222) to assume a generally flattened configuration, thereby providing end effector (200) in a non-expanded state. Moreover, when end effector (200) is pressed against an anatomical structure (e.g., the wall of a pulmonary vein, the wall of an atrium or other chamber of the heart (H), etc.), the anatomical structure may urge the contacting portion of end effector (200) to deform, such that the contacting regions of strips (220, 222) may generally conform to the surface geometry of the anatomical structure against which strips (220, 222) are pressed. Such contacting regions of strips (220, 222) may provide the substantially square spacing of electrode pairs (230) in strips (220, 222) that are contacting the tissue of the anatomical structure. In some cases (e.g., in an atrium or other chamber of the heart (H)), the tissue-contacting regions of strips (220, 222) will be substantially flat. In other cases (e.g., in a pulmonary vein), the tissue-contacting regions of strips (220, 222) may be curved. These scenarios may nevertheless still provide substantially square spacing of electrode pairs (230) in strips (220, 222) that are contacting the tissue of the anatomical structure. It should therefore be understood that the terms "substantially square configuration" and "substantially square spacing" do not necessarily require that the electrode pairs (230) at issue must be equidistantly spaced apart from each other by the same distance in two perpendicular directions that are straight. Square-spaced electrode pairs (230) may be equidistantly spaced apart from each other by the same distance in two perpendicular directions that are curved. In any event, those skilled in the art will appreciate that the substantially square spacing or substantially square configuration of electrode pairs (230) allows end effector (200) to pick up signals traveling across cardiac tissue in more directions than might otherwise be possible with electrodes having some other kind of spacing or arrangement.

In an example of a method where end effector (200) is used in the patient (PA), the physician (PH) may initially insert catheter (120) into a major vein or artery (e.g., the femoral artery) and then advance catheter (120) to position end effector (200) in the heart (H) of the patient (200). During this advancement of catheter (120), console (12) may track the position of catheter (120) in real time based on signals from position sensor (127) and render the real-time position on display (18), such that the physician (PH) may observe display (18) to determine the real-time position of catheter (120) (e.g., in relation to a preoperatively obtained image of the patient (PA)). Outer sheath (122) may be in the distal position (FIG. 2A) during the advancement of catheter (120) into the patient (PA).

Once the distal end of catheter (120) is suitably positioned, the physician (PH) may retract outer sheath (122) proximally to uncover end effector (200). End effector (200) may then reach the expanded state as shown in FIG. 2B. With end effector (200) in the expanded state, the physician (PH) may press end effector (200) against anatomical structures in the cardiac system of the patient (PA), such as the wall of the pulmonary vein, the wall of an atrium or other chamber of the heart (H), etc. As end effector (200) engages these anatomical structures, electrode pairs (230) that are in contact with the tissue will pick up signals from the tissue, thereby enabling console (12) to perform EP mapping. Console (12) may correlate the signals from electrode pairs (230) with contemporaneous signals from position sensor (127) to thereby enable storage of the precise locations of aberrant electrical signals within the cardiac tissue of the patient (PA). Console (12) may thus enable mapping of aberrant electrical signal sites with respect to a preoperatively obtained image of the cardiac anatomy of the patient (PA). With such a map being established, the physician (PH) may then provide precisely targeted ablation (e.g., using end effector (200) or some other device) or some other kind of targeted therapy based on the map.

While only EP mapping electrodes (232, 234) are shown on each electrode assembly (210), other versions of electrode assembly (210) may include ablation electrodes in addition to, or in lieu of, including EP mapping electrodes (232, 234) on electrode assembly (210). Such ablation electrodes may be used to apply RF energy to tissue that is in contact with the ablation electrodes, to thereby ablate the tissue. Each ablation electrode may be coupled with a corresponding trace or other electrical conduit on electrode assembly (210), thereby enabling console (12) to communicate RF energy through electrical conduits (not shown) in catheter (120) to the traces or other conduits on electrode assembly (210) to reach the ablation electrodes. Each electrode assembly (210) may also include one or more position sensors like position sensor (127) described above, in addition to or in lieu of second inner shaft (126) including position sensor (127).

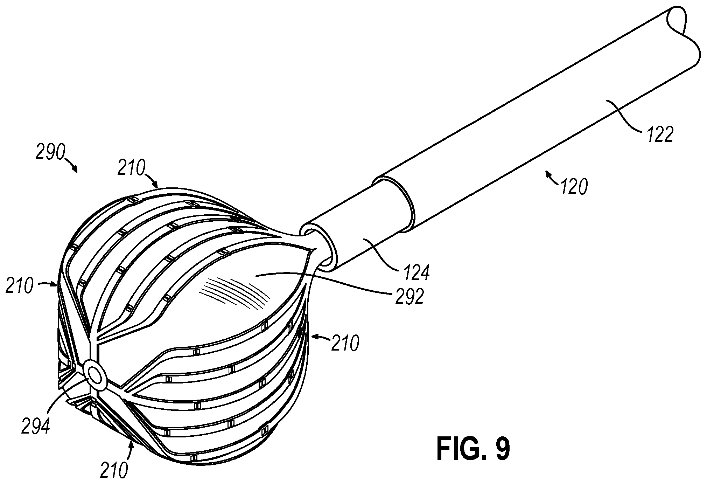

FIG. 9 shows an example of an alternative end effector (290) that is substantially similar to end effector (200) as described above. However, instead of relying on resilience of electrode assemblies (210) or some relative movement between inner shafts (124, 126) in order to transition end effector (290) from a non-expanded state to an expanded state, end effector (290) of this example includes an inflatable balloon (292). Electrode assemblies (210), which are otherwise configured and operable like electrode assemblies (210) of end effector (200), are secured to balloon (292). A hub (294) joins electrode assemblies (210) together at the distal end of balloon (292). When balloon (292) is inflated, electrode assemblies (210) are driven outwardly to the expanded state shown in FIG. 9. In some such versions, balloon (292) may include openings to allow the communication of irrigation fluid out through balloon (292), while still allowing balloon (292) to achieve an inflated state. Other suitable ways in which an end effector (290) may incorporate a balloon (292) for expansion will be apparent to those skilled in the art in view of the teachings herein.

### IV. Examples of Alternative Electrode Assemblies

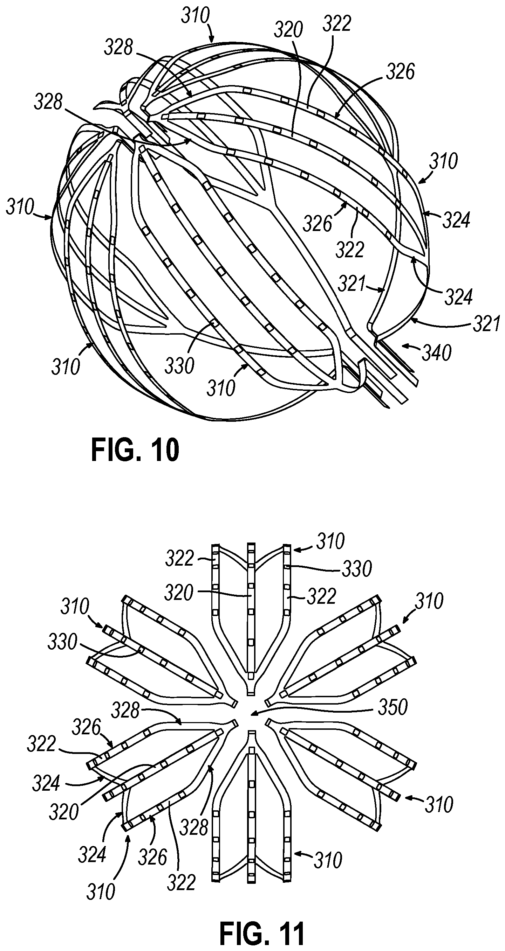

FIGS. 10-11 show examples of alternative electrode assemblies (310) that may be incorporated into end effector (200) in place of electrode assemblies (210) described above. While FIGS. 10-11 show six electrode assemblies (310), any other suitable number of electrode assemblies (310) may be incorporated into a variation of end effector (200). Except as otherwise noted below, electrode assemblies (310) of this example may be configured and operable just like electrode assemblies (210). Electrode assemblies (310) of this example include proximal portions (340) that are configured to extend proximally into the interior of first inner shaft (124), thereby proximally anchoring each electrode assembly (310) in first inner shaft (124). Distal portions (350) of electrode assemblies (310) extend proximally into the interior of second inner shaft (126), thereby distally anchoring each electrode assembly (310) in second inner shaft (126).

Each electrode assembly (310) of this example further includes a central strip (320) and a set of laterally positioned strips (322) positioned on each side of central strip (320). Each strip (322) includes a proximal segment (324), an intermediate segment (326), and a distal segment (328). Proximal segments (324) diverge outwardly, away from central strip (320). Intermediate segments (326) are parallel with central strip (320). Distal segments (328) converge inwardly, toward central strip (320). In each electrode assembly (310), a segment (310) extends between the set of strips (320, 322) and proximal portion (340) of electrode assembly (310). With strips (320, 322) being spaced apart from each other, and with electrode assemblies (310) being spaced apart from each other, a version of end effector (200) that incorporates electrode assemblies (310) is configured to allow blood to flow through end effector (200) during use of end effector (200) (e.g., when end effector (200) is in an expanded state within a chamber of the heart (H), the pulmonary vein, etc.). As noted above, such flowing blood may also reach reference electrodes (128) on second inner shaft (126) during use of end effector (200).

Each strip (320, 322) has several electrodes (330) positioned along the length of strip (320, 322). Electrodes (330) are operable to provide unipolar or bipolar EP mapping by picking up electrocardiogram signals from tissue as is known in the art. In some other variations, an electrode pair (e.g., similar to electrode pair (230) described above) is positioned where each electrode (330) is positioned on electrode assembly (310). In either case, signals picked up by electrodes (330) may be communicated back through electrical conduits (not shown) in catheter (120) to console (12), which may process the signals to provide EP mapping to thereby identify locations of aberrant electrical activity within the cardiac anatomy. This may in turn allow the physician (PH) to identify the most appropriate regions of cardiac tissue to ablate (e.g., with RF energy, cryoablation, etc.), to thereby prevent or at least reduce the communication of aberrant electrical activity across the cardiac tissue.

Each electrode assembly (310) of the present example is in the form of a flex circuit. For instance, each electrode assembly (310) may include a substrate formed of polyimide, polyether ether ketone, or any other suitable flex circuit substrate; with electrodes (330) and associated conductive traces printed on the substrate. In versions where each electrode assembly (310) is resiliently biased to bow outwardly to provide end effector (200) in an expanded configuration, one or more resilient elements may be integrated into teach electrode assembly (310). By way of example only, one or more strips of resilient material (e.g., nitinol, etc.) may be integrated into teach electrode assembly (310).

By way of example only, electrodes (330) may be formed of platinum, gold, or any other suitable material. Electrodes (330) may include various coatings, if desired. For instance, electrodes (330) may include a coating that is selected to improve the signal-to-noise ratio of signals from electrodes (330). Such coatings may include, but need not be limited to, iridium oxide (IrOx) coating, poly(3,4-ethylenedioxythiophene) (PEDOT) coating, Electrodeposited Iridium Oxide (EIROF) coating, Platinum Iridium (PtIr) coating, or any other suitable coating. Various suitable kinds of coatings that may be used for electrodes (330) will be apparent to those skilled in the art in view of the teachings herein.

In the present example electrodes (330) may be provided on the substrate of electrode assembly (210) as a thin film through a physical vapor deposition (PVD) process. way of example only, such a PVD process may be carried out in accordance with at least some of the teachings of International Patent Pub. No. WO 2015/117908, entitled "Medical Device for Ablating Tissue Cells and System Comprising a Device of This Type," published August 13, 2015, the disclosure of which is incorporated by reference herein, in its entirety; at least some of the teachings of German Patent Pub. No. 102017130152, entitled "Method for Operating a Multi-Layer Structure," published January 3, 2019, the disclosure of which is incorporated by reference herein, in its entirety; or at least some of the teachings of U.S. Pat. No. 10,061,198, entitled "Method for Producing a Medical Device or a Device with Structure Elements, Method for Modifying the Surface of a Medical Device or of a Device with Structure Elements, Medical Device and Laminated Composite with a Substrate," published August 28, 2018, the disclosure of which is incorporated by reference herein, in its entirety. Other methods may also be employed to provide electrodes (330), conductive traces, or other circuit components on electrode assemblies (310), including but not limited to sputter deposition, chemical vapor deposition (CVD), thermal deposition, etc. Regardless of the methods used, each electrode assembly (310) may ultimately constitute a flex circuit.

As noted above, each electrode assembly (310) of the present example is flexible enough to compress within outer sheath (122) as shown in FIG. 2A; yet is resiliently biased toward the outwardly bowed configuration shown in FIG. 2B. In some variations, this resilience is provided by the material forming the substrate of each electrode assembly (310). In some other variations, one or more resilient features (not shown) are added to the substrate of each electrode assembly (310) to impart the resilient bias. By way of example only, one or more nitinol strips or other nitinol structures may be applied to the substrate of each electrode assembly (310), such as along any one or more of strips (320, 322). Such nitinol strips may be applied using the vapor deposition process or other manufacturing techniques noted above.

As noted above, each electrode assembly (310) is flexible, such that a version of end effector (200) incorporating electrode assemblies (310) may conform to the contours and other surface geometry of cardiac tissue when end effector (200) is pressed against cardiac tissue (e.g., within a chamber of the heart (H), within the pulmonary vein, etc.). The deformation of end effector (200) may promote full contact between electrodes (330) and cardiac tissue. Such contact may be further promoted by providing a substantial number of electrodes (330) on each electrode assembly (310). Having a substantial number of electrodes (330) may enable end effector (200) to provide high density EP mapping through all four chambers of the heart (H), within the pulmonary vein, etc., as several pairs of electrodes (330) can provide electrocardiogram signal sensing at multiple regions of cardiac tissue simultaneously.

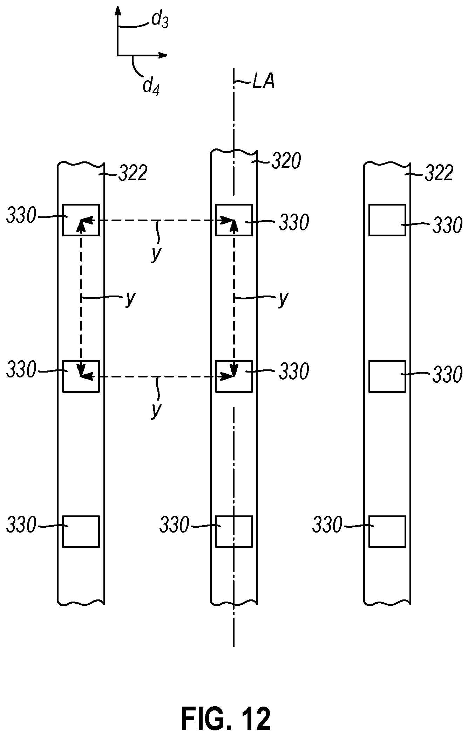

When a version of end effector (200) incorporating electrode assemblies (310) is pressed against cardiac tissue to pick up electrocardiogram signals, electrodes (330) are positioned to contact the tissue in a substantially square configuration, with substantially square spacing, as described above in the context of electrode pairs (230). This substantially square spacing is shown in FIG. 12, where the two perpendicular direction are shown as being along a first dimension (d₃) and a second dimension (d₄). As shown, a first electrode (330) on one strip (322) is spaced apart along the first dimension (d₃) from a first electrode (330) on strip (320) by a distance (y); and along the second dimension (d₄) from a second electrode (330) on strip (322) by the same distance (y). Similarly, a second electrode (330) on strip (320) is spaced apart along the first dimension (d₃) from the first pair (330) on strip (320) by that same distance (y); and along the second dimension (d₄) from the second electrode (330) on strip (222) by the same distance (y). These four electrodes (330) are thus arranged in a substantially square-shaped arrangement, with substantially square spacing, spaced equidistantly by the distance (x). This same substantially square spacing may apply to all electrodes (330) on each electrode assembly (310).

In the present example, the dimension (d₃) of spacing between electrodes (330) is parallel with the longitudinal axis (LA) of end effector (200); while the dimension (d₄) of spacing between electrodes (330) is perpendicular to the longitudinal axis (LA) of end effector (200). In some other versions, the dimensions (d₃, d₄) of spacing between electrodes (330) are obliquely oriented relative to the longitudinal axis (LA) of end effector (200), as in electrode assemblies (210).

When a version of end effector (200) that incorporates electrode assemblies (310) is pressed against an anatomical structure (e.g., the wall of a pulmonary vein, the wall of an atrium or other chamber of the heart (H), etc.), the anatomical structure may urge the contacting portion of end effector (200) to deform, such that the contacting regions of strips (320, 322) may generally conform to the surface geometry of the anatomical structure against which strips (320, 322) are pressed. Such contacting regions of strips (320, 322) may provide the substantially square spacing of electrodes (330) in strips (320, 322) that are contacting the tissue of the anatomical structure. In some cases (e.g., in an atrium or other chamber of the heart (H)), the tissue-contacting regions of strips (320, 322) will be substantially flat. In other cases (e.g., in a pulmonary vein), the tissue-contacting regions of strips (320, 322) may be curved. These scenarios may nevertheless still provide substantially square spacing of electrodes (330) in strips (320, 322) that are contacting the tissue of the anatomical structure. It should therefore be understood that the terms "substantially square configuration" and "substantially square spacing" do not necessarily require that the electrodes (330) at issue must be equidistantly spaced apart from each other by the same distance in two perpendicular directions that are straight. Square-spaced electrodes (330) may be equidistantly spaced apart from each other by the same distance in two perpendicular directions that are curved. In any event, those skilled in the art will appreciate that the substantially square spacing or substantially square configuration of electrodes (330) allows end effector (200) to pick up signals traveling across cardiac tissue in more directions than might otherwise be possible with electrodes having some other kind of spacing or arrangement.

A version of end effector (200) that incorporates electrode assemblies (310) may be used in the same manner described above with respect to a version of end effector (200) that incorporates electrode assemblies (210).

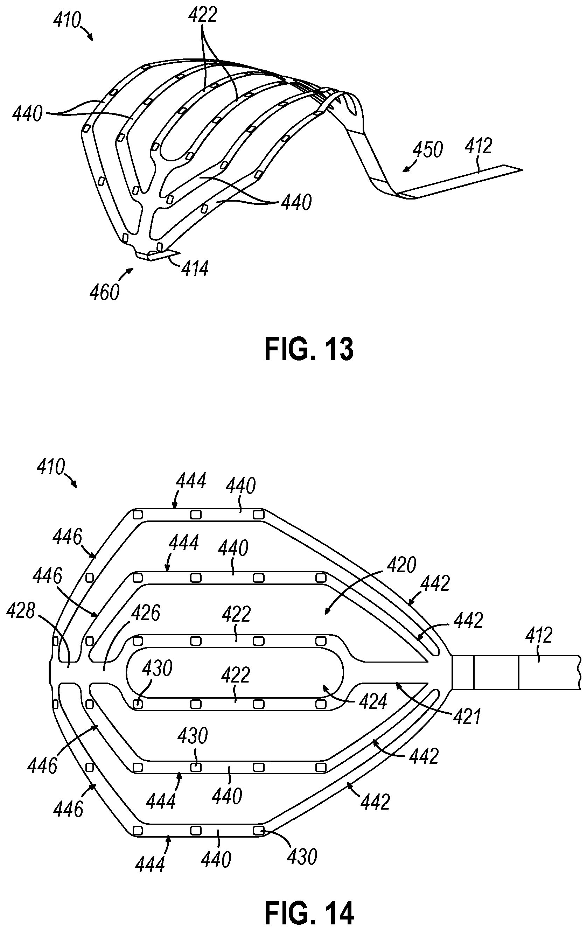

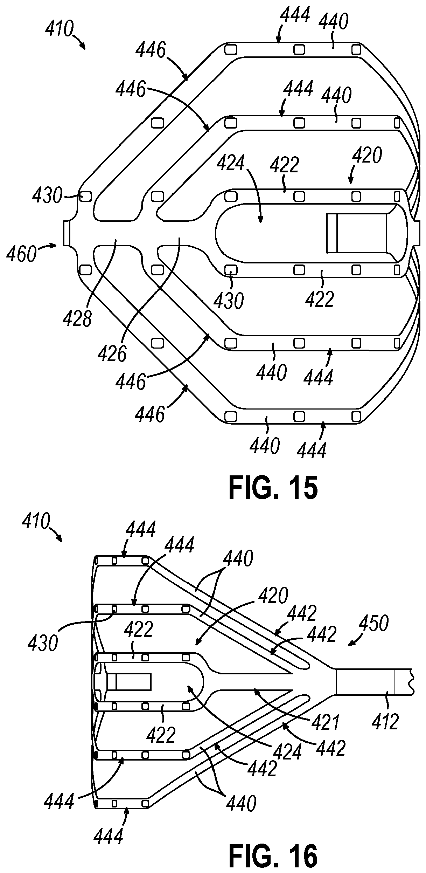

FIGS. 13-16 show an example of another alternative electrode assembly (410) that may be incorporated into end effector (200) in place of each electrode assembly (210) described above. Except as otherwise noted below, electrode assembly (410) of this example may be configured and operable just like electrode assembly (210). It should also be understood that any suitable number of electrode assemblies (410) may be incorporated into end effector (200). Electrode assemblies (410) of this example include proximal portions (450) with strips (412) that are configured to extend proximally into the interior of first inner shaft (124), thereby proximally anchoring each electrode assembly (410) in first inner shaft (124). Distal portions (460) of electrode assemblies (410) include strips (414) that are configured to extend proximally into the interior of second inner shaft (126), thereby distally anchoring each electrode assembly (410) in second inner shaft (126).

Each electrode assembly (310) of this example further includes a central portion (420) and a pair of laterally positioned strips (440) positioned on each side of central portion (420). Central portion (420) includes a proximal strip (421), a pair of laterally spaced apart intermediate strips (422), and a distal strip (426). Intermediate strips (422) are generally parallel to each other and define an oval-shaped opening (424). Each strip (440) includes a proximal segment (442), an intermediate segment (444), and a distal segment (446). Proximal segments (442) diverge outwardly. Intermediate segments (444) are parallel with each other and with intermediate strips (422). Distal segments (446) converge inwardly. Distal strip (426) extends between strips (422) and a first pair of distal segments (446). A spacing strip (428) extends between the first pair of distal segments (446) and a second pair of distal segments (446).

With strips (422, 440) being spaced apart from each other, and with electrode assemblies (410) being spaced apart from each other, a version of end effector (200) that incorporates electrode assemblies (410) is configured to allow blood to flow through end effector (200) during use of end effector (200) (e.g., when end effector (200) is in an expanded state within a chamber of the heart (H), the pulmonary vein, etc.). As noted above, such flowing blood may also reach reference electrodes (128) on second inner shaft (126) during use of end effector (200).

Each strip (422, 440) has several electrodes (430) positioned along the length of the respective strip (422, 440). Electrodes (430) are operable to provide unipolar or bipolar EP mapping by picking up electrocardiogram signals from tissue as is known in the art. In some other variations, an electrode pair (e.g., similar to electrode pair (230) described above) is positioned where each electrode (430) is positioned on electrode assembly (410). In either case, signals picked up by electrodes (430) may be communicated back through electrical conduits (not shown) in catheter (120) to console (12), which may process the signals to provide EP mapping to thereby identify locations of aberrant electrical activity within the cardiac anatomy. This may in turn allow the physician (PH) to identify the most appropriate regions of cardiac tissue to ablate (e.g., with RF energy, cryoablation, etc.), to thereby prevent or at least reduce the communication of aberrant electrical activity across the cardiac tissue.

Each electrode assembly (410) of the present example is in the form of a flex circuit. For instance, each electrode assembly (410) may include a substrate formed of polyimide, polyether ether ketone, or any other suitable flex circuit substrate; with electrodes (430) and associated conductive traces printed on the substrate. In versions where each electrode assembly (410) is resiliently biased to bow outwardly to provide end effector (200) in an expanded configuration, one or more resilient elements may be integrated into teach electrode assembly (410). By way of example only, one or more strips of resilient material (e.g., nitinol, etc.) may be integrated into teach electrode assembly (410).

By way of example only, electrodes (430) may be formed of platinum, gold, or any other suitable material. Electrodes (430) may include various coatings, if desired. For instance, electrodes (430) may include a coating that is selected to improve the signal-to-noise ratio of signals from electrodes (430). Such coatings may include, but need not be limited to, iridium oxide (IrOx) coating, poly(3,4-ethylenedioxythiophene) (PEDOT) coating, Electrodeposited Iridium Oxide (EIROF) coating, Platinum Iridium (PtIr) coating, or any other suitable coating. Various suitable kinds of coatings that may be used for electrodes (430) will be apparent to those skilled in the art in view of the teachings herein.

In the present example electrodes (430) may be provided on the substrate of electrode assembly (210) as a thin film through a physical vapor deposition (PVD) process. way of example only, such a PVD process may be carried out in accordance with at least some of the teachings of International Patent Pub. No. WO 2015/117908, entitled "Medical Device for Ablating Tissue Cells and System Comprising a Device of This Type," published August 13, 2015, the disclosure of which is incorporated by reference herein, in its entirety; at least some of the teachings of German Patent Pub. No. 102017130152, entitled "Method for Operating a Multi-Layer Structure," published January 3, 2019, the disclosure of which is incorporated by reference herein, in its entirety; or at least some of the teachings of U.S. Pat. No. 10,061,198, entitled "Method for Producing a Medical Device or a Device with Structure Elements, Method for Modifying the Surface of a Medical Device or of a Device with Structure Elements, Medical Device and Laminated Composite with a Substrate," published August 28, 2018, the disclosure of which is incorporated by reference herein, in its entirety. Other methods may also be employed to provide electrodes (430), conductive traces, or other circuit components on electrode assemblies (410), including but not limited to sputter deposition, chemical vapor deposition (CVD), thermal deposition, etc. Regardless of the methods used, each electrode assembly (410) may ultimately constitute a flex circuit.

As noted above, each electrode assembly (410) of the present example is flexible enough to compress within outer sheath (122) as shown in FIG. 2A; yet is resiliently biased toward the outwardly bowed configuration shown in FIG. 2B. In some variations, this resilience is provided by the material forming the substrate of each electrode assembly (410). In some other variations, one or more resilient features (not shown) are added to the substrate of each electrode assembly (410) to impart the resilient bias. By way of example only, one or more nitinol strips or other nitinol structures may be applied to the substrate of each electrode assembly (410), such as along any one or more of strips (422, 440). Such nitinol strips may be applied using the vapor deposition process or other manufacturing techniques noted above.

As noted above, each electrode assembly (410) is flexible, such that a version of end effector (200) incorporating electrode assemblies (410) may conform to the contours and other surface geometry of cardiac tissue when end effector (200) is pressed against cardiac tissue (e.g., within a chamber of the heart (H), within the pulmonary vein, etc.). The deformation of end effector (200) may promote full contact between electrodes (430) and cardiac tissue. Such contact may be further promoted by providing a substantial number of electrodes (430) on each electrode assembly (410). Having a substantial number of electrodes (430) may enable end effector (200) to provide high density EP mapping through all four chambers of the heart (H), within the pulmonary vein, etc., as several pairs of electrodes (430) can provide electrocardiogram signal sensing at multiple regions of cardiac tissue simultaneously.

When a version of end effector (200) incorporating electrode assemblies (410) is pressed against cardiac tissue to pick up electrocardiogram signals, electrodes (430) are positioned to contact the tissue in a substantially square configuration, with substantially square spacing, as described above in the context of electrodes (330). In particular, electrodes (430) may contact tissue in the same kind of arrangement shown in FIG. 12 and described above in the context of electrodes (330). As noted above, this substantially square spacing of electrodes (430) may provide a substantially square-shaped arrangement, with electrodes (430) being spaced equidistantly by the distance (x) along two dimensions (d₃, d₄). As also noted above, the dimension (d₃) of spacing between electrodes (430) is parallel with the longitudinal axis (LA) of end effector (200); while the dimension (d₄) of spacing between electrodes (430) is perpendicular to the longitudinal axis (LA) of end effector (200). In some other versions, the dimensions (d₃, d₄) of spacing between electrodes (430) are obliquely oriented relative to the longitudinal axis (LA) of end effector (200), as in electrode assemblies (210).

When a version of end effector (200) that incorporates electrode assemblies (410) is pressed against an anatomical structure (e.g., the wall of a pulmonary vein, the wall of an atrium or other chamber of the heart (H), etc.), the anatomical structure may urge the contacting portion of end effector (200) to deform, such that the contacting regions of strips (433, 440) may generally conform to the surface geometry of the anatomical structure against which strips (433, 440) are pressed. Such contacting regions of strips (433, 440) may provide the substantially square spacing of electrodes (430) in strips (433, 440) that are contacting the tissue of the anatomical structure. In some cases (e.g., in an atrium or other chamber of the heart (H)), the tissue-contacting regions of strips (433, 440) will be substantially flat. In other cases (e.g., in a pulmonary vein), the tissue-contacting regions of strips (433, 440) may be curved. These scenarios may nevertheless still provide substantially square spacing of electrodes (430) in strips (433, 440) that are contacting the tissue of the anatomical structure. It should therefore be understood that the terms "substantially square configuration" and "substantially square spacing" do not necessarily require that the electrodes (430) at issue must be equidistantly spaced apart from each other by the same distance in two perpendicular directions that are straight. Square-spaced electrodes (430) may be equidistantly spaced apart from each other by the same distance in two perpendicular directions that are curved. In any event, those skilled in the art will appreciate that the substantially square spacing or substantially square configuration of electrodes (430) allows end effector (200) to pick up signals traveling across cardiac tissue in more directions than might otherwise be possible with electrodes having some other kind of spacing or arrangement.

A version of end effector (200) that incorporates electrode assemblies (410) may be used in the same manner described above with respect to a version of end effector (200) that incorporates electrode assemblies (210).

### V. Examples of Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An apparatus comprising: (a) a catheter shaft assembly having a proximal end and a distal end, the catheter shaft assembly defining a longitudinal axis, the catheter shaft assembly including an outer sheath with a distal end; and (b) an end effector associated with the distal end of the catheter shaft assembly, the end effector comprising: (i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and (ii) a plurality of electrodes positioned on at least some of the strips, the electrodes being positioned relative to each other such that groups of four of the electrodes define a generally substantially square configuration.

### Example 2

The apparatus of Example 1, the end effector further comprising a plurality of electrode assemblies, each electrode assembly including a set of strips of the plurality of strips and a corresponding set of electrodes of the plurality of electrodes.

### Example 3

The apparatus of Example 2, the end effector defining a longitudinal axis, the electrode assemblies being angularly spaced apart from each other about the longitudinal axis.

### Example 4

The apparatus of any one or more of Examples 2 through 3, each set of strips comprising a plurality of strips that are parallel to each other.

### Example 5

The apparatus of any one or more of Examples 2 through 4, each set of strips comprising a plurality of strips having a plurality of segments, the plurality of segments including: (i) a first segment distally diverging outwardly away from a central axis, (ii) a second segment distally extending longitudinally from the first segment in parallel with the central axis, and (iii) a third segment distally extending longitudinally from the second segment converging inwardly toward the central axis.

### Example 6

The apparatus of Example 5, each second segment including at least some of the electrodes.

### Example 7

The apparatus of any one or more of Examples 5 through 6, each third segment including at least some of the electrodes.

### Example 8

The apparatus of any one or more of Examples 5 through 7, each set of strips further comprising a central strip, the central strip being parallel with the second segment of the other strips of the corresponding set of strips.

### Example 9

The apparatus of any one or more of Examples 5 through 7, each set of strips further comprising a central portion, the central portion comprising a pair of segments that are parallel with the second segment of the other strips of the corresponding set of strips, the central portion further defining an opening between the pair of segments of the central portion.

### Example 10

The apparatus of Example 9, the opening being oval-shaped.

### Example 11

The apparatus of any one or more of Examples 2 through 10, each electrode assembly further including a distal anchor member and a proximal anchor member, each electrode assembly being secured to the catheter shaft assembly via the distal and proximal anchor members.

### Example 12

The apparatus of Example 11, the catheter shaft assembly further comprising a first shaft and a second shaft extending distally relative to the first shaft.

### Example 13

The apparatus of Example 12, the proximal anchor member of each electrode assembly being secured to the first shaft.

### Example 14

The apparatus of any one or more of Examples 12 through 13, the distal anchor member of each electrode assembly being secured to the second shaft.

### Example 15

The apparatus of any one or more of Examples 12 through 14, the distal anchor member comprising a strip of the plurality of strips.

### Example 16

The apparatus of any one or more of Examples 12 through 15, the proximal anchor member comprising a strip of the plurality of strips.

### Example 17

The apparatus of any one or more of Examples 12 through 16, the second shaft including at least one reference electrode.

### Example 18

The apparatus of Example 17, the at least one reference electrode having an annular shape, the at least on electrode being coaxially positioned along the second shaft.

### Example 19

The apparatus of any one or more of Examples 1 through 18, each group of four electrodes including: (A) a first electrode, (B) a second electrode spaced apart from the first electrode along a first dimension by a first distance, (C) a third electrode spaced apart from the second electrode along a second dimension by the first distance, the second dimension being perpendicular to the first dimension, and (D) a fourth electrode spaced apart from the third electrode along the first dimension by the first distance, the fourth electrode being further spaced apart from the first electrode along the second dimension by the first distance.

### Example 20

The apparatus of Example 19, the first and second electrodes being positioned along a longitudinal axis, the first dimension being parallel with the longitudinal axis, the second dimension being perpendicular to the longitudinal axis.

### Example 21

The apparatus of Example 19, the first and third electrodes being positioned along a longitudinal axis, the first dimension being obliquely oriented relative to the longitudinal axis, the second dimension being obliquely oriented relative to the longitudinal axis.

### Example 22

The apparatus of any one or more of Examples 19 through 20, the first and second electrodes being positioned along a first strip of the plurality of strips.

### Example 23

The apparatus of Example 22, the third and fourth electrodes being positioned along a second strip of the plurality of strips.

### Example 24

The apparatus of Example 23, the second strip being positioned adjacent to and parallel with the first strip.

### Example 25

The apparatus of any one or more of Examples 19 or 21, the first and third electrodes being positioned along a first strip of the plurality of strips.

### Example 26

The apparatus of Example 25, the second electrode being positioned on a second strip of the plurality of strips.

### Example 27

The apparatus of Example 26, the second strip being positioned adjacent to and parallel with the first strip.

### Example 28

The apparatus of any one or more of Examples 26 through 27, the fourth electrode being positioned on a third strip of the plurality of strips.

### Example 29

The apparatus of Example 28, the third strip being positioned adjacent to and parallel with the first strip.

### Example 30

The apparatus of any one or more of Examples 28 through 29, the first strip being laterally positioned between the second and third strips.

### Example 31

The apparatus of any one or more of Examples 1 through 30, the electrodes being arranged in bipolar pairs, the bipolar pairs being positioned relative to each other such that groups of four of the bipolar pairs define a substantially square configuration.

### Example 32

The apparatus of any one or more of Examples 1 through 31, the end effector in the second configuration being configured to define a bulbous configuration.

### Example 33

The apparatus of any one or more of Examples 1 through 32, the outer sheath being operable to translate relative to the end effector between a first longitudinal position and a second longitudinal position, the outer sheath being configured to contain the end effector in the first longitudinal position, the outer sheath being configured to expose the end effector in the second longitudinal position.

### Example 34

The apparatus of any one or more of Examples 1 through 33, the end effector being operable to translate relative to the outer sheath between a first longitudinal position and a second longitudinal position, the end effector being configured to be contained in the outer sheath in the first longitudinal position, the end effector being configured to be exposed from the outer sheath in the second longitudinal position.

### Example 35

The apparatus of any one or more of Examples 1 through 34, the strips being resiliently biased to expand outwardly away from the longitudinal axis in the second configuration in response to being exposed distally relative to the distal end of the outer sheath.

### Example 36

The apparatus of Example 35, each strip including at least one resilient feature integrated into or secured to the flexible body.

### Example 37

The apparatus of Example 36, the at least one resilient feature comprising nitinol.

### Example 38

The apparatus of any one or more of Examples 1 through 37, the end effector further comprising a balloon, the balloon being operable to urge the strips to the second configuration.

### Example 39

The apparatus of Example 38, the strips being secured to the balloon.

### Example 40

The apparatus of any one or more of Examples 1 through 39, the electrodes comprising at least one pair of bipolar sensing electrodes configured to sense potentials in tissue.

### Example 41

The apparatus of any one or more of Examples 1 through 40, the electrodes comprising at least one ablation electrode.

### Example 42

The apparatus of any one or more of Examples 1 through 41, further comprising a position sensor, the position sensor being operable to generate a signal indicative of a position of one or both of at least a portion of the catheter shaft assembly or at least a portion of the end effector in three-dimensional space.

### Example 43

The apparatus of Example 42, the position sensor being located in the end effector.

### Example 44

The apparatus of Example 42, the position sensor being located on one of the strips.

### Example 45

The apparatus of Example 44, further comprising a plurality of position sensors, each strip having at least one of the position sensors integrated into the strip.

### Example 46

An apparatus comprising: (a) a catheter shaft assembly having a proximal end and a distal end, the catheter shaft assembly defining a longitudinal axis, the catheter shaft assembly including an outer sheath with a distal end; and (b) an end effector associated with the distal end of the catheter shaft assembly, the end effector comprising: (i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and (ii) a plurality of electrodes positioned on at least some of the strips, the electrodes being positioned relative to each other such that groups of four of the electrodes each include: (A) a first electrode, (B) a second electrode spaced apart from the first electrode along a first dimension by a first distance, (C) a third electrode spaced apart from the second electrode along a second dimension by the first distance, the second dimension being perpendicular to the first dimension, and (D) a fourth electrode spaced apart from the third electrode along the first dimension by the first distance, the fourth electrode being further spaced apart from the first electrode along the second dimension by the first distance.

### Example 47

An apparatus comprising: (a) a catheter shaft assembly having a proximal end and a distal end, the catheter shaft assembly defining a longitudinal axis, the catheter shaft assembly including an outer sheath with a distal end; and (b) an end effector associated with the distal end of the catheter shaft assembly, the end effector comprising: (i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and (ii) a plurality of electrode pairs positioned on at least some of the strips, the electrode pairs being positioned relative to each other such that groups of four of the electrode pairs define a substantially square configuration.

### Example 48

An apparatus comprising: (a) a catheter shaft assembly having a proximal end and a distal end, the catheter shaft assembly defining a longitudinal axis, the catheter shaft assembly including an outer sheath with a distal end; and (b) an end effector associated with the distal end of the catheter shaft assembly, the end effector comprising: (i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and (ii) a plurality of electrodes positioned on at least some of the strips, the electrodes being positioned relative to each other such that electrodes within groups of four of the electrodes define substantially square spacing between the electrodes in each group of four.

### Example 49

An apparatus comprising: (a) a catheter shaft assembly having a proximal end and a distal end, the catheter shaft assembly defining a longitudinal axis, the catheter shaft assembly including an outer sheath with a distal end; and (b) an end effector associated with the distal end of the catheter shaft assembly, the end effector comprising: (i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and (ii) a plurality of electrodes positioned on at least some of the strips, the electrodes being spaced apart from each other by the same distance in two perpendicular directions.

### VI. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

It should be understood that any of the examples described herein may include various other features in addition to or in lieu of those described above. By way of example only, any of the examples described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

Embodiments:
1. An apparatus comprising:
   (a) a catheter shaft assembly having a proximal end and a distal end, the catheter shaft assembly defining a longitudinal axis, the catheter shaft assembly including an outer sheath with a distal end; and
   (b) an end effector associated with the distal end of the catheter shaft assembly, the end effector comprising:
      (i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and
      (ii) a plurality of electrodes positioned on at least some of the strips, the electrodes being positioned relative to each other such that groups of four of the electrodes define a generally substantially square configuration.
2. The apparatus of embodiment 1, the end effector further comprising a plurality of electrode assemblies, each electrode assembly including a set of strips of the plurality of strips and a corresponding set of electrodes of the plurality of electrodes.
3. The apparatus of embodiment 2, the end effector defining a longitudinal axis, the electrode assemblies being angularly spaced apart from each other about the longitudinal axis.
4. The apparatus of embodiment 2, each set of strips comprising a plurality of strips that are parallel to each other.
5. The apparatus of embodiment 1, each group of four electrodes including:
   (A) a first electrode,
   (B) a second electrode spaced apart from the first electrode along a first dimension by a first distance,
   (C) a third electrode spaced apart from the second electrode along a second dimension by the first distance, the second dimension being perpendicular to the first dimension, and
   (D) a fourth electrode spaced apart from the third electrode along the first dimension by the first distance, the fourth electrode being further spaced apart from the first electrode along the second dimension by the first distance.
6. The apparatus of embodiment 5, the first and second electrodes being positioned along a longitudinal axis, the first dimension being parallel with the longitudinal axis, the second dimension being perpendicular to the longitudinal axis.
7. The apparatus of embodiment 5, the first and third electrodes being positioned along a longitudinal axis, the first dimension being obliquely oriented relative to the longitudinal axis, the second dimension being obliquely oriented relative to the longitudinal axis.
8. The apparatus of embodiment 5, the first and second electrodes being positioned along a first strip of the plurality of strips.
9. The apparatus of embodiment 8, the third and fourth electrodes being positioned along a second strip of the plurality of strips.
10. The apparatus of embodiment 5, the first and third electrodes being positioned along a first strip of the plurality of strips.
11. The apparatus of embodiment 10, the second electrode being positioned on a second strip of the plurality of strips.
12. The apparatus of embodiment 11, the fourth electrode being positioned on a third strip of the plurality of strips.
13. The apparatus of embodiment 1, the electrodes being arranged in bipolar pairs, the bipolar pairs being positioned relative to each other such that groups of four of the bipolar pairs define a substantially square configuration.
14. The apparatus of embodiment 1, the strips being resiliently biased to expand outwardly away from the longitudinal axis in the second configuration in response to being exposed distally relative to the distal end of the outer sheath.
15. The apparatus of embodiment 14, each strip including at least one resilient feature integrated into or secured to the flexible body.
16. The apparatus of embodiment 1, the end effector further comprising a balloon, the balloon being operable to urge the strips to the second configuration.
17. The apparatus of embodiment 1, the electrodes comprising at least one pair of bipolar sensing electrodes configured to sense potentials in tissue.
18. The apparatus of embodiment 1, the electrodes comprising at least one ablation electrode.
19. An apparatus comprising:
   (a) a catheter shaft assembly having a proximal end and a distal end, the catheter shaft assembly defining a longitudinal axis, the catheter shaft assembly including an outer sheath with a distal end; and
   (b) an end effector associated with the distal end of the catheter shaft assembly, the end effector comprising:
      (i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and
      (ii) a plurality of electrodes positioned on at least some of the strips, the electrodes being positioned relative to each other such that groups of four of the electrodes each include:
         (A) a first electrode,
         (B) a second electrode spaced apart from the first electrode along a first dimension by a first distance,
         (C) a third electrode spaced apart from the second electrode along a second dimension by the first distance, the second dimension being perpendicular to the first dimension, and
         (D) a fourth electrode spaced apart from the third electrode along the first dimension by the first distance, the fourth electrode being further spaced apart from the first electrode along the second dimension by the first distance.
20. An apparatus comprising:
   (a) a catheter shaft assembly having a proximal end and a distal end, the catheter shaft assembly defining a longitudinal axis, the catheter shaft assembly including an outer sheath with a distal end; and
   (b) an end effector associated with the distal end of the catheter shaft assembly, the end effector comprising:
      (i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and
      (ii) a plurality of electrodes positioned on at least some of the strips, the electrodes being spaced apart from each other by the same distance in two perpendicular directions.

## Claims

1. An apparatus comprising:
(a) a catheter shaft assembly having a proximal end and a distal end, the catheter shaft assembly defining a longitudinal axis, the catheter shaft assembly including an outer sheath with a distal end; and
(b) an end effector associated with the distal end of the catheter shaft assembly, the end effector comprising:
(i) a plurality of strips, the strips being configured to transition between a first configuration and a second configuration, the strips being configured to fit within the outer sheath in the first configuration, the strips being configured to expand outwardly away from the longitudinal axis in the second configuration when exposed distally relative to the distal end of the outer sheath, and
(ii) a plurality of electrodes positioned on at least some of the strips, the electrodes being positioned relative to each other such that groups of four of the electrodes define a generally substantially square configuration.

2. The apparatus of claim 1, wherein the end effector comprises a first electrode assembly (210) comprising a proximal end strip (212), a distal end strip (214), and a first group of the plurality of strips that extend between the proximal end strip and the distal end strip.

3. The apparatus of claim 2, wherein the first group of the plurality of strips includes a central strip (220) and a plurality of laterally positioned strips (222) positioned on each side of the central strip.

4. The apparatus of claim 2 or claim 3, wherein the proximal end strip extends proximally into the interior of a first inner shaft, thereby proximally anchoring the first electrode assembly in the first inner shaft (124), and wherein the distal end strip extends proximally into the interior of a second inner shaft (126) and is secured to a movable actuator via the second inner shaft.

5. The apparatus of any of claims 2 to 4, comprising second, third, and fourth electrode assemblies that are the same as the first electrode assembly, wherein each electrode assembly is connected to a different movable actuator.

6. The apparatus of claim 1, each group of four electrodes including:
(A) a first electrode,
(B) a second electrode spaced apart from the first electrode along a first dimension by a first distance,
(C) a third electrode spaced apart from the second electrode along a second dimension by the first distance, the second dimension being perpendicular to the first dimension, and
(D) a fourth electrode spaced apart from the third electrode along the first dimension by the first distance, the fourth electrode being further spaced apart from the first electrode along the second dimension by the first distance.

7. The apparatus of claim 6, the first and second electrodes being positioned along a longitudinal axis, the first dimension being parallel with the longitudinal axis, the second dimension being perpendicular to the longitudinal axis.

8. The apparatus of claim 6, the first and third electrodes being positioned along a longitudinal axis, the first dimension being obliquely oriented relative to the longitudinal axis, the second dimension being obliquely oriented relative to the longitudinal axis.

9. The apparatus of claim 6, the first and second electrodes being positioned along a first strip of the plurality of strips, the third and fourth electrodes being positioned along a second strip of the plurality of strips.

10. The apparatus of claim 6, the first and third electrodes being positioned along a first strip of the plurality of strips, the second electrode being positioned on a second strip of the plurality of strips, the fourth electrode being positioned on a third strip of the plurality of strips.

11. The apparatus of claim 1, the electrodes being arranged in bipolar pairs, the bipolar pairs being positioned relative to each other such that groups of four of the bipolar pairs define a substantially square configuration.

12. The apparatus of claim 1, the strips being resiliently biased to expand outwardly away from the longitudinal axis in the second configuration in response to being exposed distally relative to the distal end of the outer sheath, and optionally wherein each strip including at least one resilient feature integrated into or secured to the flexible body.

13. The apparatus of claim 1, the end effector further comprising a balloon, the balloon being operable to urge the strips to the second configuration.

14. The apparatus of claim 1, the electrodes comprising at least one pair of bipolar sensing electrodes configured to sense potentials in tissue.

15. The apparatus of claim 1, the electrodes comprising at least one ablation electrode.
